# EUROPEAN PATENT SPECIFICATION

(11) **EP 2 517 615 B1**
(45) Date of publication and mention of the grant of the patent: **12.08.2015**
(21) Application number: 11803460.2
(22) Date of filing: 24.06.2011
(51) Int. Cl.: A61B 1/00, G02B 23/24, A61B 1/005

(54) **ENDOSCOPE APPARATUS WITH MULTIPLE LAYER ENDOSCOPE TUBE**
ENDOSKOPVORRICHTUNG MIT MEHRSCHICHTIGEM ENDOSKOPROHR
DISPOSITIF ENDOSCOPIQUE AVEC TUBE D'ENDOSCOPE MULTICOUCHES

(30) Priority: 05.07.2010 JP 2010153467
(43) Date of publication of application: 31.10.2012
(73) Proprietor: OLYMPUS MEDICAL SYSTEMS CORP., Tokyo 151-0072 (JP)
(72) Inventor: TAKEUCHI Yasuo, Tokyo 151-0072 (JP)
(74) Representative: Schicker, Silvia
(86) International application number: PCT/JP2011/064579
(87) International publication number: WO 2012/005124

(56) References cited:
- JP-A- 2002 360 504
- JP-A- 2006 218 157
- JP-A- 2007 289 467
- JP-A- 2008 054 786
- JP-U- H0 223 502
- JP-U- H02 118 502
- US-A1- 2007 255 105

## Description

### Technical Field

The present invention relates to a tube provided in an endoscope apparatus including an insertion portion to be inserted into a body, and in particular, to an endoscope tube provided in the insertion portion, and also relates to an endoscope apparatus.

### Background Art

As is well known, an endoscope apparatus of a medical instrument includes an image pickup apparatus being image pickup means, and is introduced into a body cavity of a patient to perform a variety of examinations and treatments for an affected area in the body using an observation image photographed by the image pickup apparatus. Such endoscope apparatuses include one that introduces an insertion portion into an oral cavity, an anus, and a urethra entrance to observe the interior of tube cavities and tubes in a body, specifically, a digestive system such as an esophagus, a gaster, a large intestine, and a duodenum, a urinary system such as a urethra, a ureter, and a urinary bladder, or a respiratory system such as a trachea and lungs, and one that punctures a body wall near the umbilical region with a needle, and pushes the needle through the wall to introduce an insertion portion into an abdominal cavity.

The insertion portion of such an endoscope apparatus includes a treatment instrument insertion channel through which a variety of treatment instruments are inserted in and extracted from a treatment instrument insertion port (also referred to as the treatment instrument insertion and withdrawal aperture) provided at an operation portion and are taken in and from a distal end of the insertion portion in order to treat a lesion and perform cytodiagnosis in which tissue of a lesion is abrased or harvested to examine a mucous membrane and a secreting fluid.

Such treatment instrument insertion channels include one that is disclosed in Japanese Patent Application Laid-Open Publication No. 5-95898. The disclosed treatment instrument insertion channel is composed of a flexible tube obtained by embedding a net in which metal fibers are plaited in a tube made of a polymeric material. The Patent Literature 1 discloses a technique that performs multiple spot welding on a region close to an end portion at a side of a connection portion to a distal end rigid portion of the insertion portion in the net in order to protect a connection part to the distal end rigid portion of the treatment instrument insertion channel so as to prevent the connection part from being buckled and deformed.

In the conventional treatment instrument channel, a part around the area connected to the distal end rigid portion is hardened to prevent problems such as buckling and deformation.

However, the conventional treatment instrument channel has a problem that a tube part with which the treatment instrument inserted trough the treatment instrument insertion port is first brought into contact is hard to disperse a contact load by the treatment instrument because of the metal net embedded and therefore tends to be eroded and tends to make a hole by abrasion, damage or the like.

Inside the operation portion of the endoscope apparatus there is provided a metal part communicating with the treatment instrument insertion opening and this part is connected to the treatment instrument insertion channel. In the conventional treatment instrument insertion channel, the vicinity of a connection portion connected with the metal part is the part with which the treatment instrument is first brought into contact, and there has been a problem that this part tends to be subjected to abrasion, damage or the like.

In addition, a load is exerted not only to the treatment instrument insertion channel but also to various types of tubes disposed inside the endoscope such as an air and water supply channel from a washing instrument such as a washing blush which is inserted into the tubes in washing the endoscope, and there is a case that the tubes are subjected to abrasion, damage or the like.

Further, in the conventional tube such as the treatment instrument insertion channel, since the connection end portion to be connected with the connection part by covering the part is hard to be deformed because of the metal net, there is also a problem that the connection with the part is difficult. Therefore, the conventional treatment instrument insertion channel provided in the endoscope apparatus has a problem that operability in assemble with the part to be connected is lowered.

Thus, the present invention has been made in view of the above problems and an object of the present invention is to provide a tube which is provided in an endoscope apparatus, has an improved durability by reducing a load by contact with a treatment instrument which is inserted into the tube to suppress abrasion, damage or the like, and has good operability in assemble with the connection part.

US 2007/255105 A1 and JP 2008 054786 A disclose several-examples of endoscopes and treatment tool insertion channels which are divided into a flexible portion and a rigid portion.

### Disclosure of Invention

### Means for Solving the Problem

The present invention is defined in claim 1. A tube provided in an endoscope apparatus according to an aspect of the present invention includes: a first area provided inside a rigid region in the endoscope apparatus; and a second area provided inside a flexible region in the endoscope apparatus, wherein a rigidity of the first area is set lower than a rigidity of the second area.

In addition, an endoscope apparatus according to an aspect of the present invention has a distal end and a proximal end, and includes: a treatment instrument channel or an air and water supply tube having a first area provided as being inserted in a rigid part which is provided on a proximal end side, and a second area provided as being inserted in a flexible part which is provided on a distal end side, the first area having a rigidity lower than a rigidity of the second area; and a block body provided in the rigid part, the block body having a first opening provided at a position inclined toward a rear side with a predetermined angle with respect to a longitudinal axis and a second opening communicating with the first opening formed at a front, and provided with a connection portion to which an end portion of the second area of the treatment instrument channel or the air and water supply tube is outwardly inserted and connected.

Further, an endoscope apparatus according to another aspect of the present invention includes: a tube comprising a first area provided inside a rigid region and a second area provided inside a flexible region, a rigidity of the first area being set lower than a rigidity of the second area; and a block body provided in the rigid part, the block body having a first opening provided at a position inclined toward a rear side with a predetermined angle with respect to a longitudinal axis and the second opening communicating with the first opening formed at a front, and provided with a connection portion to which an end portion of the first area of the tube is outwardly inserted and connected.

### Brief Description of the Drawings

Fig. 1 is a diagram relating to an embodiment of the present invention and showing a configuration of an endoscope apparatus.
Fig. 2 is a cross-sectional view relating to the embodiment of the present invention and showing an internal configuration of an operation portion.
Fig. 3 is a cross-sectional view relating to the embodiment of the present invention and showing an internal configuration of a distal end portion.
Fig. 4 is a partial cross-sectional view relating to the embodiment of the present invention and showing a configuration of a treatment instrument insertion channel.
Fig. 5 is a diagram relating to the embodiment of the present invention and showing a configuration of a blade layer of a first modified example.
Fig. 6 is a diagram relating to the embodiment of the present invention and showing a configuration of a blade layer of a second modified example.
Fig. 7 is a diagram relating to the embodiment of the present invention and showing a configuration of a blade layer of a third modified example.
Fig. 8 is a cross-sectional view relating to the embodiment of the present invention and taken along line VIII-VIII in Fig. 4.
Fig. 9 is a cross-sectional view of the operation portion relating to the embodiment of the present invention and showing a state in which a high frequency coagulator being a treatment instrument for an endoscope is inserted in the treatment instrument insertion channel.
Fig. 10 is a perspective view relating to the embodiment of the present invention and illustrating connection between the treatment instrument insertion channel and a connection ring.
Fig. 11 is a cross-sectional view relating to the embodiment of the present invention and showing an internal configuration of an operation portion of a modified example.
Fig. 12 is a partial cross-sectional view relating to the embodiment of the present invention and showing a configuration of a treatment instrument insertion channel of a modified example.
Fig. 13 is a partial cross-sectional view relating to the embodiment of the present invention and showing a configuration of a treatment instrument insertion channel of a modified example different from that in Fig. 12.
Fig. 14 is a partial cross-sectional view relating to the embodiment of the present invention and showing an internal configuration of an insertion portion of a modified example.

### Best Mode for Carrying Out the Invention

Hereinafter, an embodiment of an endoscope apparatus of the present invention will be described with reference to the drawings. It should be noted that in the following descriptions, the drawings according to each embodiment are schematic representations, so that a relationship between a thickness and a width of each part, and a thickness ratio of each part are different from actual ones. A dimension and a ratio of one diagram may be different from those of another diagram.

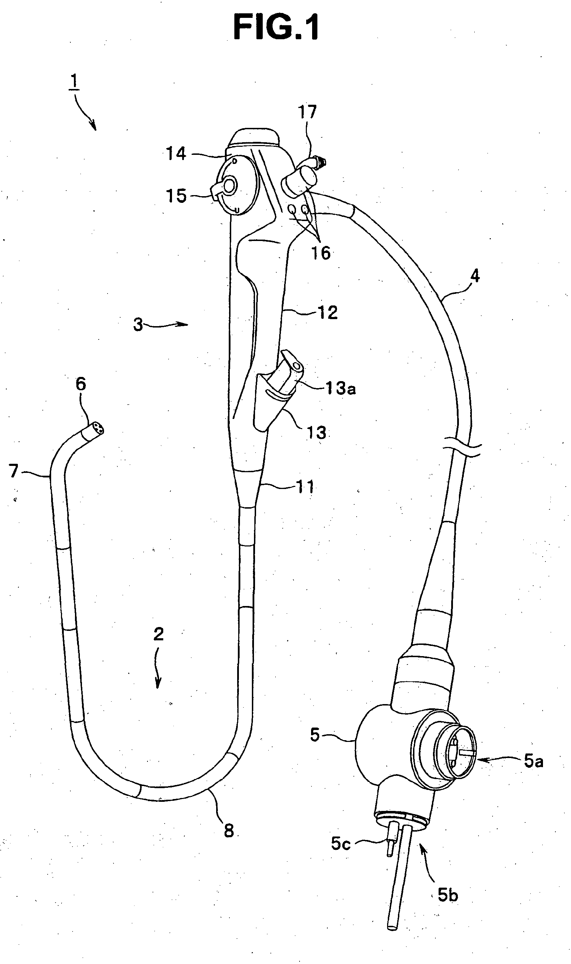

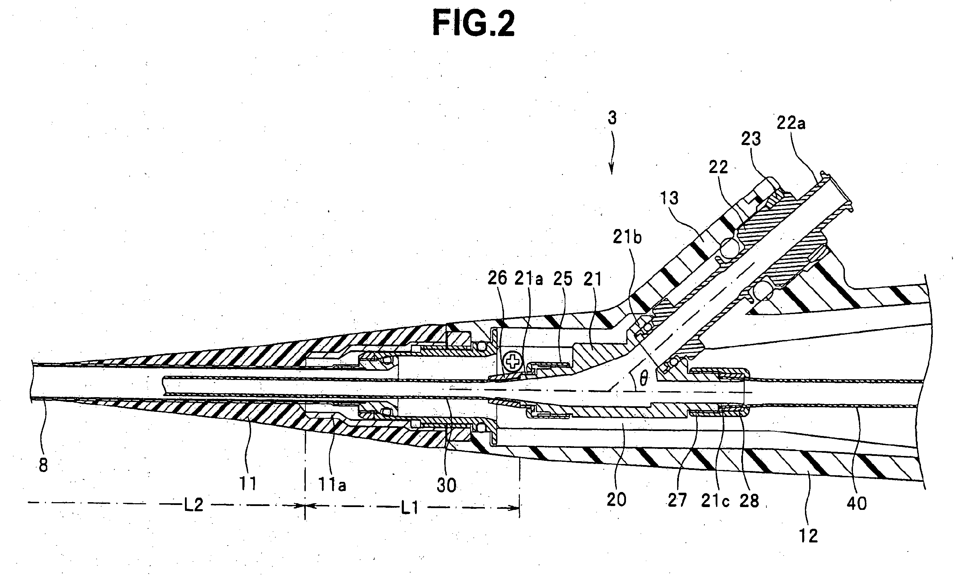

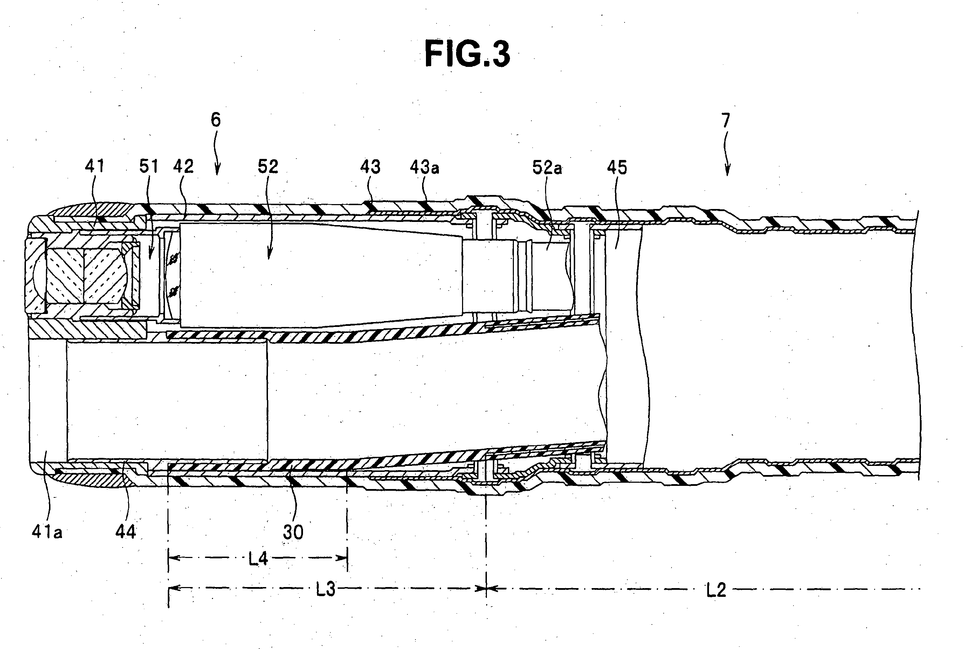

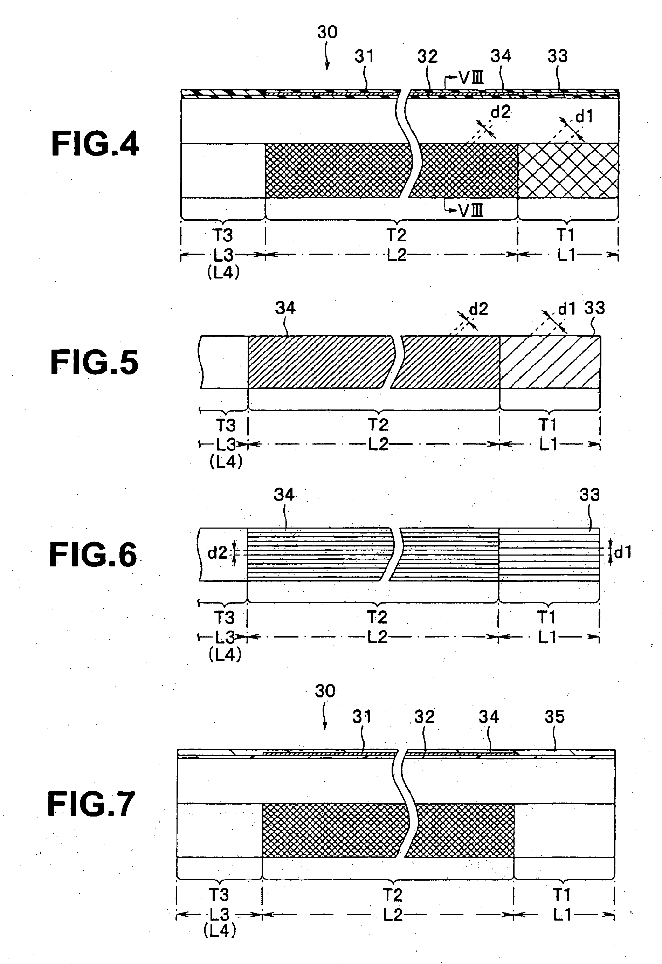

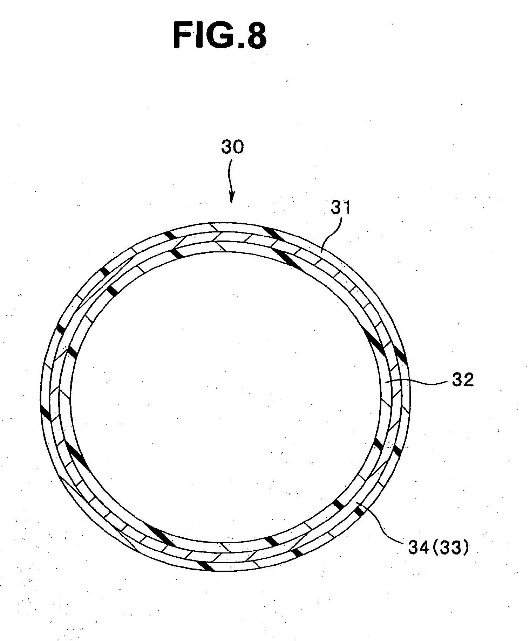

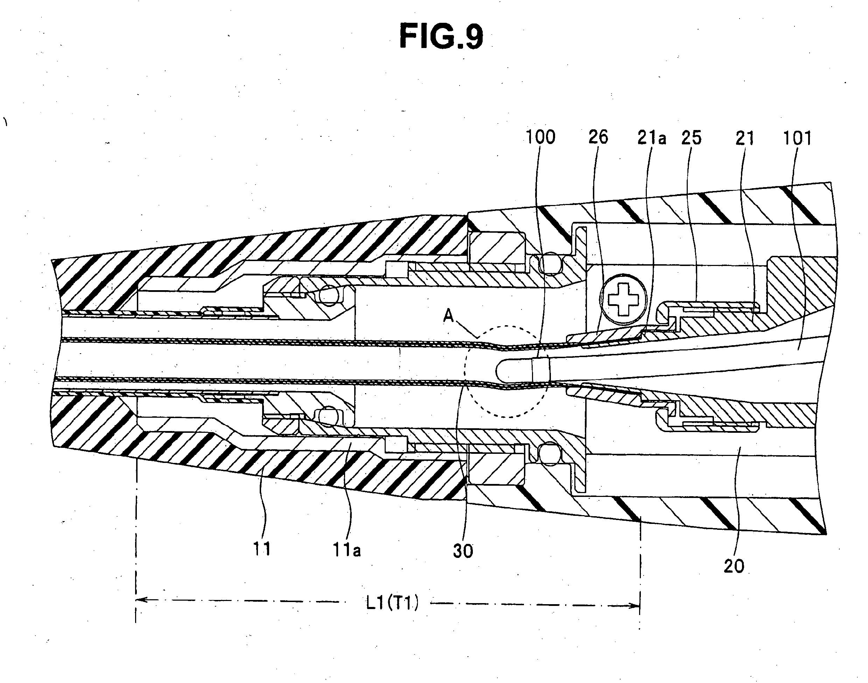

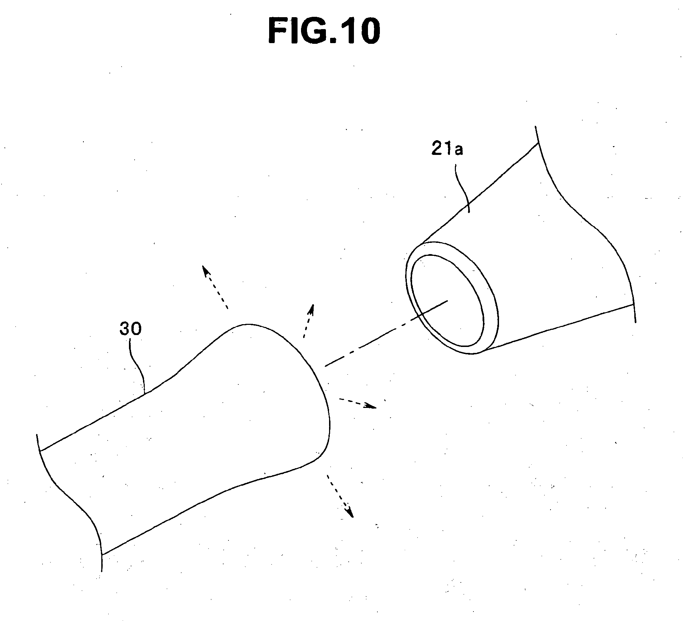

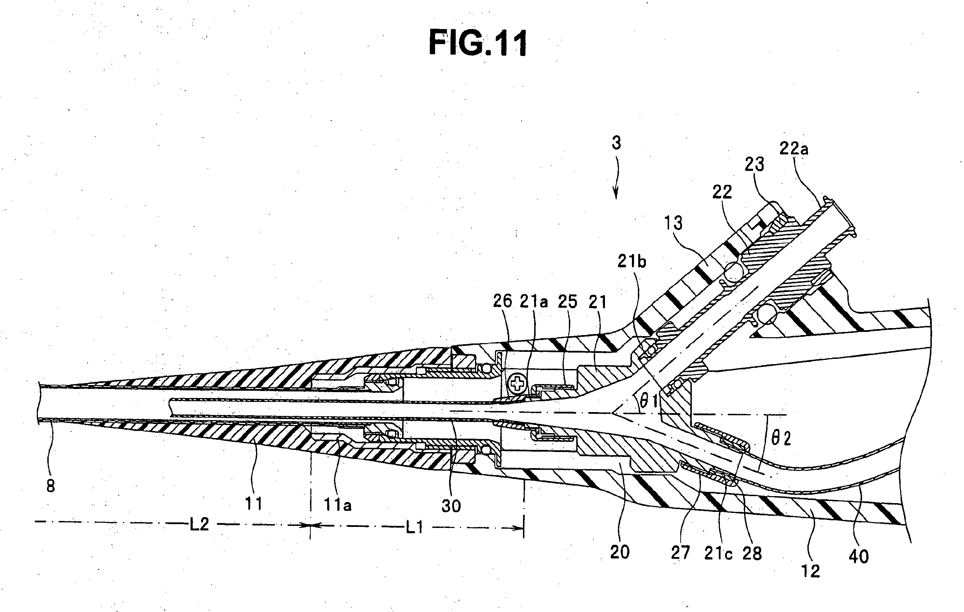

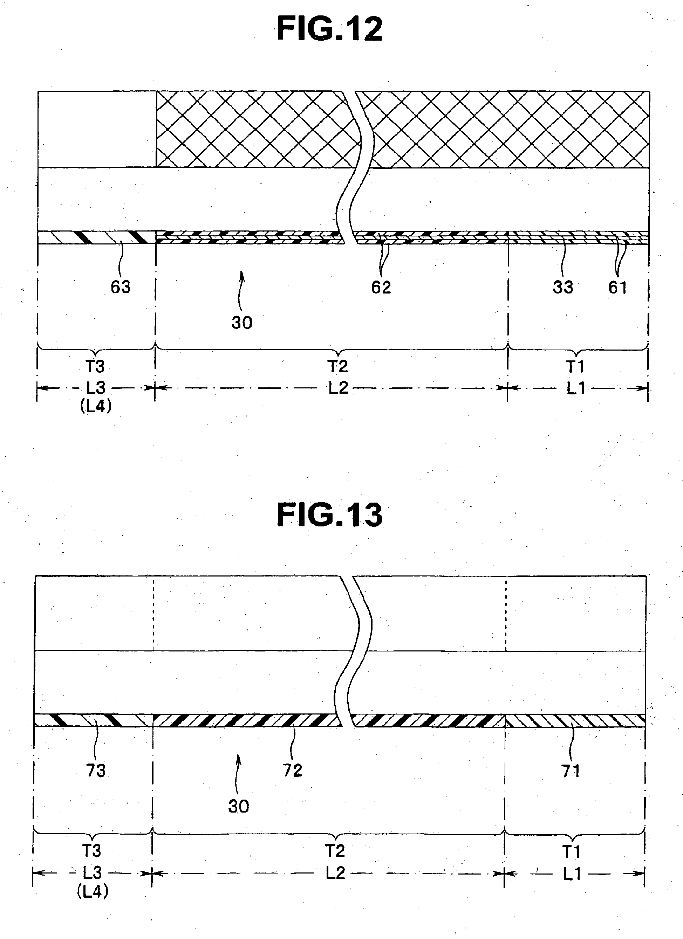

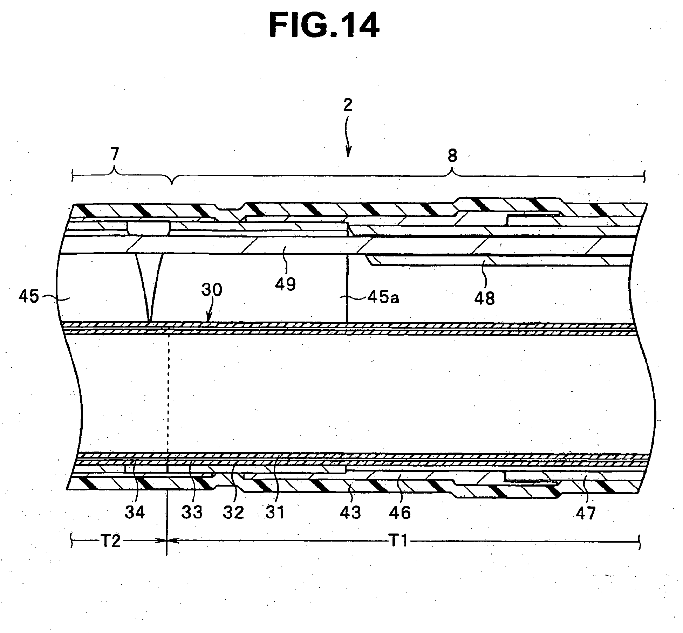

Fig. 1 through Fig. 7 relate to an embodiment of the present invention. Fig. 1 showing a configuration of an endoscope apparatus, Fig. 2 is a cross-sectional view showing an internal configuration of an operation portion, Fig. 3 is a cross-sectional view showing an internal configuration of a distal end portion, and Fig. 4 is a partial cross-sectional view showing a configuration of a treatment instrument insertion channel. Fig. 5 through Fig. 7 are diagrams showing configurations of blade layers of modified examples. Fig. 8 is a cross-sectional view taken along line VIII-VIII in Fig. 4, Fig. 9 is a cross-sectional view of the operation portion showing a state in which a high frequency coagulator being a treatment instrument for an endoscope is inserted in the treatment instrument insertion channel, Fig. 10 is a perspective view illustrating connection between the treatment instrument insertion channel and a connection ring, Fig. 11 is a cross-sectional view showing an internal configuration of an operation portion of a modified example, Fig. 12 is a partial cross-sectional view showing a configuration of a treatment instrument insertion channel of a modified example, Fig. 13 is a partial cross-sectional view showing a configuration of a treatment instrument insertion channel of a modified example different from that in Fig. 12, and Fig. 14 is a partial cross-sectional view showing an internal configuration of an insertion portion of a modified example.

An endoscope apparatus 1 shown in Fig. 1 includes an insertion portion 2, an operation portion 3 connected with a proximal end of the insertion portion 2, a universal cord 4 being a composite cable extended from the operation portion 3, and an endoscope connector 5 provided at an end portion of the universal cord.

The insertion portion 2 is a flexible tube body in which a distal end portion 6, a bending portion 7, and a flexible tube portion 8 are linked in this order from a distal end (front). The operation portion 3 includes in the following order from the distal end (front): a bend preventing portion 11 connected so as to cover a proximal end portion of the flexible tube portion 8; a grasping portion 12 including a treatment instrument insertion portion 13 in which an exchangeable forceps plug 13a detachably provided in a treatment instrument insertion port described later is provided; and a main operation portion 14 in which a bending lever 15, a plurality of switches 16 for performing operations of air supply, water supply, and suction, or a variety of optical system operations carried out by image pickup means and illumination means provided at the distal end portion 6, and a detachably exchangeable suction valve 17 are provided.

The distal end portion 6 of the insertion portion 2 incorporates an image pickup device of a CCD or a CMOS for photographing tissue of a body, and image signals photoelectrically converted by an image pickup unit described later are outputted to a video processor (not shown) detachably connected with the endoscope connector 5 via the universal cord 4. The endoscope connector 5 includes on a side surface an electrical connector portion 5a with which a connector of a video converter being an electrical cable not shown and connected with a connector portion of the video processor (not shown) is connected, a light source connector portion 5b inserted into a connector portion of a light source apparatus not shown in which an illuminating light source is incorporated extends from a proximal end. Also, the video processor is connected with a monitor (not shown) that displays an endoscope image.

In the present embodiment, a light guide not shown is inserted from the distal end portion 6 of the insertion portion 2 to the universal cord 4. The light guide guides illuminating light from a light source of the light source apparatus, so that the subject is irradiated with the transmitted illuminating light from the distal end portion 6. Moreover, a plug 5c for air supply and water supply is provided at the light source connector portion 5b of the endoscope connector 5.

Further, in the endoscope apparatus 1 of the present embodiment, the bending portion 7 bends in two directions of up and down, and a swivel operation of the bending lever 15 provided at the operation portion 3 bends the bending portion 7 up and down. It should be noted that in the endoscope apparatus 1, the bending direction of the bending portion 7 is not limited to the two directions of up and down, and the bending portion 7 may bend in four directions including left and right (in a circumferential direction around an axis depending on operations of up and down, left and right).

Now, the internal configuration of the operation portion 3 will be described below with reference to Fig. 2.

In the grasping portion 12 of the operation portion 3, a fixing plate 20 is provided, and a branch component 21 is fixed to the fixing plate 20.

The branch component 21 includes: at a front side, a front tube connection portion 21a which is connected with a tube 30 being a treatment instrument insertion channel of the present embodiment and has an opening; at a side of the treatment instrument insertion portion 13, a shaft connection portion 21b which is connected with a plug shaft 22 and has an opening; and at a rear side, a rear tube connection portion 21c which is connected with a tube 40 for air supply, water supply, or suction and has an opening. The branch component 21 is a metal block body that has an opening formed on each of the front and rear end portions in a direction along an axis of the operation portion 3, and a part inclined with respect to a longitudinal axis by a predetermined angle θ toward the treatment instrument insertion portion 13.

The plug shaft 22 is a substantially annular metal member and inserted in the treatment instrument insertion portion 13 and fixed by the fixing ring 23 near the opening of the treatment instrument insertion portion 13 so that the treatment instrument insertion port 22a for which the forceps plug 13a is a detachable pipe sleeve protrudes. Further, an end portion of the plug shaft 22 in the operation portion 3 is inserted and connected to the shaft connection portion 21b.

The front tube connection portion 21 a is tapered so as to reduce a diameter of an end portion and is fitted on the tube 30 for the insertion of the treatment instrument so that the tube 30 is brought into intimate contact with a circumference surface of the front tube connection portion 21a. In addition, a tightening ring 25 inserted into the tube 30 in advance is screwed with the front tube connection portion 21a. Depending on a degree of screw tightness between the front tube connection portion 21a and the tightening ring 25, an inward flange is brought into contact with an outward flange of a holding ring 26 inserted into the tube 30 in advance to move the holding ring 26 to the rear side. Thereby, the holding ring 26 holds the tube 30 by pressing the tube 30 on the front tube connection portion 21a.

Also, the rear tube connection portion 21c is tapered so as to reduce a diameter of an end portion and is fitted on the tube 40 for air supply, water supply, or suction so that the tube 40 is brought into intimate contact with a circumference surface of the rear tube connection portion 21c. Further, the rear tube connection portion 21c is also screwed with a tightening ring 27 inserted in the tube 40 in advance. The tightening ring 27 is provided so as to include a holding ring 28, and depending on a degree of screw tightness between the rear tube connection portion 21 c and the tightening ring 27, an end portion of the holding ring 28 is brought into contact with an inward flange to move the tightening ring 27 with the holding ring 28 toward the front side. Thereby, the holding ring 28 holds the tube 40 by pressing the tube 40 on the rear tube connection portion 21 c.

The tube 30 for the insertion of the treatment instrument is inserted through the bend preventing portion 11 of the operation portion 3 and the substantially total length of the insertion portion 2 from the flexible tube portion 8 to the distal end portion 6. Further, the tube 40 for air supply, water supply, or suction is inserted through the suction valve 17 provided at the main operation portion 14 of the operation portion 3 and the universal cord 4 connected with the operation portion 3, and branches in the operation portion 3 to be connected with the plug 5c for air supply, water supply and the like.

An annular connection component 11a forming a metal component used to be connected and fixed to the grasping portion 12 is provided inside the rear part of the bend preventing portion 11. Thus, in the bend preventing portion 11, a part positioned ahead of the connection component 11a is more flexible. That is, in the operation portion 3, the grasping portion 12 including the treatment instrument insertion portion 13 and the part having the connection component 11a of the bend preventing portion 11 are rigid. Further, the flexible tube portion 8 of the insertion portion 2 includes a tubular blade formed by winding a thin board spirally to set a predetermined flexibility.

As is apparent from the foregoing, the tube 30 for the insertion of the treatment instrument of the present embodiment is inserted from the connection position with the front tube connection portion 21a to the rigid part of the operation portion 3 by a predetermined length L1, as well as the tube 30 is inserted from the part of the bend preventing portion 11 where the connection component 11a is not provided in the operation portion 3 to the part of the insertion portion 2 having the flexibility due to the flexible tube portion 8 by a predetermined length L2. As described later, the part of the predetermined length L2 includes the flexible bending portion 7 of the insertion portion 2, and the length L2 is up to the distal end portion 6.

Now, the internal configuration of the distal end portion 6 of the endoscope apparatus 1 will be briefly described below with reference to Fig. 3.

The distal end portion 6 includes a distal end rigid portion 41 that is a block body made of metal, non-metal (e.g., resin), or a non-metal mixture component (a formed material or a composite from resin and metal) and a metal rigid pipe 42 fitted to a rear part of the distal end rigid portion 41, and the distal end rigid portion 41 and the rigid pipe 42 are integrally covered by an outer cover 43 to form the distal end portion 6.

A lens unit 51 having a lens holding frame in which a plurality of optical systems are provided is fitted to the distal end rigid portion 41. An image pickup unit 52 is provided at a rear part of the lens unit 51. A cable 52a for transferring control signals such as image pickup signals extends from the image pickup unit 52.

A metal net pipe 43a provided on an inner surface of the outer cover 43 is fastened to a rear outer circumferential portion of the rigid pipe 42, and a bending piece 45 is pivotably provided at a rear end. The plurality of bending pieces 45 are pivotably connected in the bending portion 7.

Further, the distal end rigid portion 41 has an opening 41a at a distal end. A tube connection pipe 44 is inserted and fixed to the opening 41a. The tube connection pipe 44 is connected so that the tube 30 for the insertion of the treatment instrument covers a rear outer circumferential portion of the tube connection pipe 44.

The bending portion 7 forms the flexible part in the insertion portion 2 in which the circumferences of the pivotably linked bending pieces 45 are covered by the outer cover 43 including the net pipe 43a.

As seen from the foregoing description, the tube 30 for the insertion of the treatment instrument of the present embodiment is, as described above, inserted from the part of the bend preventing portion 11 positioned ahead of the connection component 11a in the operation portion 3 to the bending portion 7 through the flexible tube portion 8 by the predetermined length L2, as well as the tube 30 is inserted from the connection position with the tube connection pipe 44 to the bending portion 7 by a predetermined length L3 in the rigid distal end portion 6. It should be noted that the tube 30 for the insertion of the treatment instrument may also be inserted in the rigid distal end portion 6 by a predetermined length L4 as shown in Fig. 3, being a length from a connection position of the tube connection pipe 44 to the connection portion to the distal end rigid portion 41.

Next, the configuration of the tube 30 being a treatment instrument insertion channel of the present embodiment will be described with reference to Figs. 4 and 5.

As shown in Figs. 4 and 5, the tube 30 for the insertion of the treatment instrument has a three-layer tube structure in which a plurality of blade layers 33 and 34 are linked in a part of a longitudinal direction, and the blade layers 33 and 34 are net layers formed by weaving element wires being fibers such as metal in a plurality of resin layers 31 and 32. The present embodiment adopts a multiple layer tube structure, illustrating the two resin layers 31 and 32, and the two blade layers 33 and 34.

Specifically, in an area (first area) T1 of the tube 30 having the above-described predetermined length L1 and provided from the connection position with the front tube connection portion 2 1 a to the rigid part of the operation portion 3, the low-density blade layer 33 is provided between the two resin layers 31 and 32, the blade layer 33 being low-density net layer (low-density element wire layer) in which element wires formed of metal are woven into a loose lattice. It should be noted that the low-density blade layer 33 is not limited to metal, and carbonaceous fibers such as acrylic fibers, non-metal such as resin, or non-metal mixture components (e.g., resin mixed metal) may also be plaited into the low-density blade layer 33. Also, the layer 33 may not be plaited into a lattice. For example, as shown in Fig. 5, the layer 33 may be wound into a loose spiral (diagonally wound) and provided between the two resin layers 31 and 32, or as shown in Fig. 6, the layer 33 may be loosely arranged in column and provided between the two resin layers 31 and 32.

Further, in an area (second area) T2 of the tube 30 having the predetermined length L2 and provided at a flexible part from the part of the bend preventing portion 11 positioned ahead of the connection component 11a of the operation portion 3 to the bending portion 7 through the flexible tube portion 8, a high-density blade layer 34 is provided between the resin layers 31 and 32, the blade layer 34 being high-density net layer (high-density element wire layer) in which the element wires made of metal are woven into a dense lattice. It should be noted that the high-density blade layer 34 is not also limited to metal, and carbonaceous fibers such as acrylic fibers, non-metal such as resin, or non-metal mixture components (e.g., resin mixed metal) may also be plaited into the high-density blade layer 34. Also, the high-density blade layer 34 may not be plaited into a lattice. For example, as shown in Fig. 5, the layer 34 may be wound into a dense spiral (diagonally wound) and provided between the two resin layers 31 and 32, or as shown in Fig. 6, the layer 34 may be densely arranged in column and provided between the two resin layers 31 and 32.

A pitch width d1 between the element wires plaited (wound or arranged) in the low-density blade layer 33 provided at the area T1 is 1.5 times or greater (d1≥d2×1.5) than a pitch width d2 between the element wires plaited (wound or arranged) in the high-density blade layer 34 provided at the area T2.

Further, in an area (a first area or a third area) T3 of the tube 30 having the predetermined length L3 or L4 and provided from the connection position with the tube connection pipe 44 in the rigid distal end portion 6 to the bending portion 7, the blade layers 33 and 34 are not provided, and the area T3 has a two-layer tube structure of a plurality of, here, the two resin layers 31 and 32.

It is desirable that a fluororesin (PTFE layer) be formed or disposed on an inner surface of the tube 30 to reduce friction with a treatment instrument for an endoscope to be inserted in the tube 30.

Thus, in the tube 30, depending on a plaited (wound or arranged) density of the element wires made of metal, non-metal, or a non-metal mixture component in the blade layers 33 and 34, the rigidity of the area T1 having the predetermined length L1 is lower than that of the area T2 having the predetermined length L2, that is, the area T1 is more flexible and deformable, and the area T3 having the predetermined length L3 or L4 in which the blade layers 33 and 34 are not provided is the most flexible (has the lowest rigidity).

In other words, in the tube 30, the area T1 from the connection position with the front tube connection portion 21a to the rigid part of the operation portion 3 has a lower predetermined rigidity than that of the area T2 from the part of the bend preventing portion 11 positioned ahead of the connection component 11a in the operation portion 3 to the bending portion 7 through the flexible tube portion 8, and the area T3 from the connection position with the tube connection pipe 44 in the rigid distal end portion 6 to the bending portion 7 is the most flexible part.

As shown in Fig. 9, in the endoscope apparatus 1 of the present embodiment described above, for example, a treatment instrument 100 for an endoscope being a high frequency coagulator is introduced from the treatment instrument insertion port 22a of the treatment instrument insertion portion 13, and inserted in the tube 30 from the branch component 21. At this time, as shown in Fig. 2, in the treatment instrument 100 for an endoscope, since an insertion path from the side of the shaft connection portion 21 b to the front tube connection portion 21 a has a predetermined angle θ, a treatment instrument insertion portion 101 with a resilient force is introduced in a bent state in the branch component 21. Therefore, the treatment instrument 100 for an endoscope has a reaction force that makes the treatment instrument insertion portion 101 be returned to a straight state.

Then, passing the branch component 21, the treatment instrument 100 for an endoscope gives stress caused by the reaction force in the treatment instrument insertion portion 101 to, in particular, the vicinity of the connection part (the area T1) with the front tube connection portion 21 a of the tube 30, and comes into contact with the inner surface of the tube 30. At this time, as shown by a broken line circle A in Fig. 9, in the tube 30, a part of the area T1 with which the treatment instrument 100 for an endoscope comes into contact is deformed to reduce a contact load made by the treatment instrument 100 for an endoscope.

That is, in the tube 30 of the present embodiment, the low-density blade layer 33 is provided and the flexible and deformable part of the area T1 having a predetermined rigidity and positioned at the rear side disperses a load of abutment (contact) with the introduced treatment instrument 100 for an endoscope, so that falling off, abrasion, and damage are unlikely to occur, and the durability is improved. Further, a cleaning brush is introduced into the tube 30, which is a treatment instrument insertion channel, at the time of cleaning/sterilization performed before and after the use of the endoscope apparatus 1, and then the brush cleaning is carried out. Therefore, as above, because the tube 30 of the present embodiment also disperses a load of abutment (contact) with the cleaning brush introduced into the part of the area T1 at the time of the cleaning/sterilization, also at this time, the part of the area T1 is unlikely to fall off and abrasion and damage are unlikely to occur, and the durability is improved.

Because the end portion at the side of the area T1 positioned at the rear of the tube 30 is more deformable than the area T2 positioned at the middle owing to the low-density blade layer 33, as shown in Fig. 10, the diameter of the opening can be easily increased. Therefore, the tube 30 can be easily inserted and connected to the front tube connection portion 21a. Thereby, the assembling property of the tube 30 to the front tube connection portion 21a is improved.

Returning to Fig. 7, the area T1 of the tube 30 may have the same tube structure as the area T3, having only a resin layer 35.

Further, in the tube 30, the high-density blade layer 34 is provided and the area T2 positioned at the middle and having a high rigidity is adapted and provided to the flexible tube portion 8 and the bending portion 7 of the insertion portion 2 that are meandered and bent as needed, as a flexible or pliant configuration. That is, in order to endure the movement of the flexible tube portion 8 and the bending portion 7 that are meandered and bent many times, the durability of the tube 30 is increased with the area T2 having a high rigidity because of the high-density blade layer 34.

Further, because the area T3 positioned at the front of the tube 30 is provided at the rigid distal end portion 6 of the insertion portion 2, the area T3 is not affected by meandering and bending, and the area T3 is unlikely to be affected by the reaction force from the treatment instrument insertion portion 101 of the treatment instrument 100 for an endoscope. Therefore, the area T3 has a plurality of, here, two layers, the resin layers 31 and 32 without the blade layers 33 and 34.

Thus, the end portion of the tube 30 at the front area T3 is deformable, and the end portion can be easily fitted over and connected to the tube connection pipe 44. Thereby, in the tube 30, the assembling property to the front tube connection portion 21a as well as the assembling property to the tube connection pipe 44 is improved. It should be noted that the area T3 of the tube 30 may be the same as the area T1, that is, a three-layer tube structure having the low-density blade layer 33.

As described in the foregoing, in the tube 30 for the insertion of the treatment instrument of the present embodiment, the two areas T1 and T3 provided at a rigid region in the endoscope apparatus 1 and the area T2 provided in the flexible region including flexibility or pliantness are set at the best rigidity (hardness) for each region.

It should be noted that the above-described configuration of the tube 30 may also be applied to the tube 40 for air supply, water supply, or suction. That is, the configuration of the tube 40 may be the same as that of the tube 30. In the endoscope apparatus 1, the operation portion 3 being the rigid region and the area provided in the endoscope connector 5 may be a multiple, for example, three-layer tube structure in which the low-density blade layer 33 is provided, or two layers, the resin layers 31 and 32. The area provided in the universal cord 4 being the flexible region may be a multiple, for example, three-layer tube structure in which the high-density blade layer 34 is provided.

Furthermore, as shown in Fig. 11, for example, in an endoscope apparatus 1 for the lower digestive tract, a branch component 21 may be a metal block body in which the side of the treatment instrument insertion portion 13 forms a predetermined angle θ1 with respect to the front and rear end portions in a direction along the axis of the operation portion 3 and the longitudinal axis. Further, the rear tube connection portion 21c extending toward the rear side has a predetermined angle θ2 with respect to the axis.

In the endoscope apparatus 1 having such a configuration, for brush cleaning, if a cleaning brush (not shown) is introduced into the tube 40 from the rear side, as described above, a load of abutment (contact) with the cleaning brush introduced into the part of the area T1 at the time of the cleaning/sterilization is also dispersed. Therefore, also at this time, the part of the area T1 of the tube 30 is unlikely to fall off and abrasion and damage are unlikely to occur, and the durability is improved.

Furthermore, as shown in Fig. 12, in the tube 30, only the low-density blade layer 33 is provided in the areas T1 and T2, and resins with different degrees of resin hardness are formed so as to cover an inner surface and an outer surface of the low-density blade layer 33 to make a multiple, here, a three-layered tube structure with a plurality of, for example, two resin layers 61 and 62. Further, in the area T3 a resin tube 63 with a lower resin hardness than that of the resin layers 61 and 62 may be linked to alter the best hardness (rigidity) depending on each of the areas T1 to T3.

Further, in the tube 30, the areas T1 and T2 are not limited to the above-described three-layered tube structure with the blade layers 33 and 34 and the two resin layers 31 and 32. As shown in Fig. 13, for example, a plurality of, here, three resin tubes 71, 72, and 73 that form the tube 30 may have the resin hardness altered depending on each of the areas T1 to T3, and the resin tubes 71, 72, and 73 may be linked with each other in a longitudinal direction to set the best rigidity (hardness) for each region (area). It should be noted that a fluororesin (PTFE, FEP, PFA, PCTFE, and the like) is formed or disposed on an inner circumferential surface of each of the resin tubes 61, 62, 63, 71, 72, and 73 to reduce friction with a treatment instrument for an endoscope to be inserted in the tube.

Furthermore, as shown in Fig. 14, the tube 30 extending in the insertion portion 2 of the endoscope apparatus 1 may be provided so that a boundary portion between the bending portion 7 and the flexible tube portion 8 in the insertion portion 2 is a boundary portion between the low-density blade layer 33 and the high-density blade layer 34. That is, the first area T1 of the tube 30 extends from the bending portion 7 of the insertion portion 2 to a proximal end side, and the second area T2 of the tube 30 extends from a distal end side of the insertion portion 2 to the bending portion 7.

In the insertion portion 2, a distal end part of a joint pipe 46 is fitted over to a most proximal end piece 45a of a plurality of bending pieces 45, and a proximal end part of the joint pipe 46 is coupled to a spiral tubular flex pipe 47 to form the flexible tube portion 8. Further, for the bending pieces 45, by pulling and releasing an angle wire 49 inserted in an angle coil 48, adjacent pieces are swiveled.

It should be noted that the foregoing embodiment has illustrated the configuration of the endoscope apparatus 1 as a flexible endoscope including the insertion portion 2 with the flexible tube portion 8, but the embodiment may be applied to the endoscope apparatus 1 as a rigid endoscope including the insertion portion 2 in which the region of the flexible tube portion 8 is composed of a rigid pipe. The described invention is not limited to the embodiments described above, and in a practical stage, a variety of changes can be made to the extent that the changes do not depart from the present invention as defined in claim 1.

The present application is filed claiming the priority of Japanese Patent Application No. 2010-153467 filed in Japan on July 5, 2010.

## Claims

1. An endoscope apparatus (1) comprising:
an insertion portion (2) which is a flexible tube body in which a distal end portion (6), a bending portion (7), and a flexible tube portion (8) are linked in this order from a distal end,
an operation portion (3) connected with a proximal end of the insertion portion (2), the operation portion (3) including in the following order from the distal end, a bend preventing portion (11) connected so as to cover a proximal end portion of the flexible tube portion (8), a grasping portion (12) including a treatment instrument insertion portion (13) and a main operation portion (14), and
an endoscope tube (30) provided from the distal end portion of the insertion portion (2) to the operation portion (3) through the bending portion (7), and connected with a branch component (21) provided in the operation portion (3),
the branch component (21) being fixed to a fixing plate (20) which is provided in the grasping portion (12) of the operation portion (3) and having a first opening provided at a position inclined toward a proximal end side with a predetermined angle (θ) with respect to a longitudinal axis and a second opening formed at a distal end and communicating with the first opening, and a third opening provided on a proximal end with respect to the longitudinal axis,
**characterized in that** the endoscope tube (30) comprises:
a first area (T1) provided in the operation portion (3) and having a multiple layer structure with an element wire layer (33) between resin layers (31, 32) provided from a connection position with the second opening to the bend preventing portion (11) of the operation portion (3); and
a second area (T2) provided from a distal end face of the bend preventing portion (11) of the operation portion (3) to the bending portion (7) through the insertion portion (2) and having a multiple layer structure with a high-density blade layer (34) being a high-density net layer provided to be continuous to the element wire layer (33) of the first area (T1) between the resin layers (31, 32),
wherein an end portion of the first area (T1) is connected with the second opening of the branch component (21), and the element wire layer (33) is provided between the resin layers (31, 32), as a low-density blade layer being a low-density net layer to reduce a rigidity of the first area (T1) to be lower than a rigidity of the second area (T2) so as to easily deform a connection part with a treatment instrument (100) inserted from the first opening.

2. The endoscope apparatus (1) according to claim 1, wherein the rigidity of the first area (T1) is set lower than the rigidity of the second area (T2) with a pitch width between element wires used to plait the low-density net layer (33) being 1.5 times or greater than a pitch width between element wires used to plait the high-density net layer (34).

3. The endoscope apparatus (1) according to claim 1, wherein the first area (T1) extends from the bending portion (7) to a proximal end side of the insertion portion (2) of the endoscope apparatus (1), and the second area (T2) extends from a distal end side of the insertion portion (2) to the bending portion (7).

4. The endoscope apparatus (1) according to claim 1, wherein the high-density blade layer (34) and the low-density blade layer (33) are metal element wires woven into a lattice.

5. The endoscope apparatus (1) according to claim 4, wherein the endoscope tube (30) is a treatment instrument channel into which the treatment instrument (100) is inserted.

6. The endoscope apparatus (1) according to any of the preceding claims, further comprising an air and water supply tube (40) connected to the third opening of the branch component (21).

## Patentansprüche

1. Endoskopgerät (1), das umfasst:
einen Einführabschnitt (2), der ein flexibler Röhrenkörper ist, in dem ein distaler Endabschnitt (6), ein Biegeabschnitt (7) und ein flexibler Röhrenabschnitt (8) in dieser Reihenfolge von einem distalen Ende aus miteinander verbunden sind,
einen Betätigungsabschnitt (3), der mit einem proximalen Ende des Einführabschnitts (2) verbunden ist, wobei der Betätigungsabschnitt (3) in der folgenden Reihenfolge von dem distalen Ende aus einen
Biegeverhinderungsabschnitt (11), der so verbunden ist, dass er einen proximalen Endabschnitt des flexiblen Röhrenabschnitts (8) überdeckt, einen Greifabschnitt (12) mit einem Behandlungsinstrumenteinführabschnitt (13) und einen Hauptbetätigungsabschnitt (14) umfasst, und
eine Endoskopröhre (30), die von dem distalen Endabschnitt des Einführabschnitts (2) zu dem Betätigungsabschnitt (3) durch den Biegeabschnitt (7) vorgesehen ist und mit einer Zweigkomponente (21) verbunden ist, die in dem Betätigungsabschnitt (3) vorgesehen ist,
die Zweigkomponente (21), die an einer in dem Greifabschnitt (12) des Betätigungsabschnitts (3) vorgesehenen Fixierungsplatte (20) fixiert ist und eine erste Öffnung, die an einer Position in Richtung einer proximalen Endseite mit einem vorbestimmten Winkel (θ) bezüglich einer Längsachse geneigt vorgesehen ist, und eine zweite Öffnung, die an einem distalen Ende ausgebildet ist und mit der ersten Öffnung kommuniziert, und eine dritte Öffnung hat, die an einem proximalen Ende bezüglich der Längsachse vorgesehen ist,
**dadurch gekennzeichnet, dass** die Endoskopröhre (30) umfasst:
ein erstes Gebiet (T1), das in dem Betätigungsabschnitt (3) vorgesehen ist und eine mehrschichtige Struktur mit einer Elementdrahtschicht (33) zwischen Harzschichten (31, 32) hat, die von einer Verbindungsposition mit der zweiten Öffnung zu dem Biegeverhinderungsabschnitt (11) des Betätigungsabschnitts (3) vorgesehen sind; und
ein zweites Gebiet (T2), das von einer distalen Endfläche des Biegeverhinderungsabschnitts (11) des Betätigungsabschnitt (3) zu dem Biegeabschnitt (7) durch den Einführabschnitt (2) vorgesehen ist und eine mehrschichtige Struktur mit einer hochdichten Schneideschicht (34) hat, die eine hochdichte Netzschicht ist, die kontinuierlich zu der Elementdrahtschicht (33) des ersten Gebiets (T1) zwischen den Harzschichten (31, 32) vorgesehen ist,
wobei ein Endabschnitt des ersten Gebiets (T1) mit der zweiten Öffnung der Zweigkomponente (21) verbunden ist und die Elementdrahtschicht (33) als eine Schneideschicht mit geringer Dichte, die eine Netzschicht mit geringer Dichte ist, zwischen den Harzschichten (31, 32) vorgesehen ist, um eine Steifigkeit des ersten Gebiets (T1) so zu reduzieren, dass sie geringer als eine Steifigkeit des zweiten Gebiets (T2) ist, um ein Verbindungsteil mit einem Behandlungsinstrument (100), das von der ersten Öffnung eingeführt ist, leicht zu deformieren.

2. Endoskopgerät (1) gemäß Anspruch 1, wobei die Steifigkeit des ersten Gebiets (T1) mit einem Abstand zwischen zum Flechten der Netzschicht (33) mit geringer Dichte verwendeten Elementdrähten, der das 1,5-fache oder mehr als ein Abstand zwischen zum Flechten der hochdichten Netzschicht (34) verwendeten Elementdrähten beträgt, so eingestellt ist, dass sie geringer als die Steifigkeit des zweiten Gebiets (T2) ist.

3. Endoskopgerät (1) gemäß Anspruch 1, wobei sich das erste Gebiet (T1) von dem Biegeabschnitt (7) zu einer proximalen Endseite des Einführabschnitts (2) des Endoskopgeräts (1) erstreckt und sich das zweite Gebiet (T2) von einer distalen Endseite des Einführabschnitts (2) zu dem Biegeabschnitt (7) erstreckt.

4. Endoskopgerät (1) gemäß Anspruch 1, wobei die hochdichte Schneideschicht (34) und die Schneideschicht (33) mit geringer Dichte Metallelementdrähte sind, die zu einem Gitter gewoben sind.

5. Endoskopgerät (1) gemäß Anspruch 4, wobei die Endoskopröhre (30) ein Behandlungselementkanal ist, in den das Behandlungsinstrument (100) eingeführt ist.

6. Endoskopgerät (1) gemäß einem der vorhergehenden Ansprüche, das ferner eine Zufuhrröhre (40) für Luft und Wasser umfasst, die mit der dritten Öffnung der Zweigkomponente (21) verbunden ist.

## Revendications

1. Appareil endoscopique (1) comprenant :
une partie (2) d'insertion qui est un corps en tube flexible dans lequel une partie (6) d'extrémité distale, une partie (7) de flexion, et une partie (8) de tube flexible sont liées dans cet ordre à partir d'une extrémité distale,
une partie (3) d'opération connectée avec une extrémité proximale de la partie (2) d'insertion, la partie (3) d'opération incluant, dans l'ordre suivant à partir de l'extrémité distale, une partie (11) de prévention de flexion connectée de façon à recouvrir une partie d'extrémité proximale de la partie (8) de tube flexible, une partie (12) de préhension incluant une partie (13) d'insertion d'instrument de traitement et une partie (14) principale d'opération, et
un tube (30) d'endoscope prévu de la partie d'extrémité distale de la partie (2) d'insertion jusqu'à la partie (3) d'opération par la partie (7) de flexion, et connecté avec un composant (21) d'embranchement prévu dans la partie (3) d'opération,
le composant (21) d'embranchement étant fixé à une plaque (20) de fixation qui est prévue dans la partie (12) de préhension de la partie (3) d'opération et ayant une première ouverture prévue en une position inclinée vers un côté d'extrémité proximale avec un angle (θ) prédéterminé par rapport à un axe longitudinal et une deuxième ouverture formée à une extrémité distale et communiquant avec la première ouverture, et une troisième ouverture prévue sur une extrémité proximale par rapport à l'axe longitudinal,
**caractérisé en ce que** le tube (30) d'endoscope comprend :
une première zone (T1) prévue dans la partie (3) d'opération et ayant une structure à couches multiples avec une couche (33) de fils élémentaires entre des couches (31, 32) de résine prévue d'une position de connexion avec la deuxième ouverture jusqu'à la partie (11) de prévention de flexion de la partie (3) d'opération ;
et
une deuxième zone (T2) prévue d'une face d'extrémité distale de la partie (11) de prévention de flexion de la partie (3) d'opération jusqu'à la partie (7) de flexion par la partie (2) d'insertion et ayant une structure à couches multiples avec une couche (34) de lame haute densité étant une couche en réseau haute densité prévue pour être continue avec la couche (33) de fils élémentaires de la première zone (T1) entre les couches (31, 32) de résine,
dans lequel une partie d'extrémité de la première zone (T1) est connectée avec la deuxième ouverture du composant (21) d'embranchement, et la couche (33) de fils élémentaires est prévue entre les couches (31, 32) de résine, comme une couche de lame basse densité étant une couche en réseau basse densité pour réduire une rigidité de la première zone (T1) afin qu'elle soit inférieure à une rigidité de la deuxième zone (T2) de façon à déformer facilement une partie de connexion avec un instrument (100) de traitement inséré par la première ouverture.

2. Appareil endoscopique (1) selon la revendication 1, dans lequel la rigidité de la première zone (T1) est fixée inférieure à la rigidité de la deuxième zone (T2) avec une largeur de pas entre fils élémentaires utilisés pour tresser la couche (33) en réseau basse densité étant 1,5 fois ou plus une largeur de pas entre fils élémentaires utilisés pour tresser la couche (34) en réseau haute densité.

3. Appareil endoscopique (1) selon la revendication 1, dans lequel la première zone (T1) s'étend de la partie (7) de flexion jusqu'à un côté d'extrémité proximale de la partie (2) d'insertion de l'appareil endoscopique (1), et la deuxième zone (T2) s'étend d'un côté d'extrémité distale de la partie (2) d'insertion jusqu'à la partie (7) de flexion.

4. Appareil endoscopique (1) selon la revendication 1, dans lequel la couche (34) de lame haute densité et la couche (33) de lame basse densité sont des fils élémentaires métalliques tissés en treillis.

5. Appareil endoscopique (1) selon la revendication 4, dans lequel le tube (30) d'endoscope est un canal d'instrument de traitement dans lequel l'instrument (100) de traitement est inséré.

6. Appareil endoscopique (1) selon l'une quelconque des revendications précédentes, comprenant en outre un tube (40) d'alimentation en air et en eau connecté à la troisième ouverture du composant (21) d'embranchement.
